**Europäisches Patentamt**

**European Patent Office**

**Office européen des brevets**

(19)

(11) Veröffentlichungsnummer: **0 445 553 A1**

# (12) EUROPÄISCHE PATENTANMELDUNG

(21) Anmeldenummer: **91101973.5**

(22) Anmeldetag: **13.02.91**

(51) Int. Cl.5: **C07D 213/85**

(30) Priorität: **09.03.90 DD 338544**

(43) Veröffentlichungstag der Anmeldung:
**11.09.91 Patentblatt 91/37**

(84) Benannte Vertragsstaaten:
**AT BE CH DE ES FR GB IT LI NL SE**

(71) Anmelder: **ARZNEIMITTELWERK DRESDEN GMBH**
**Wilhelm-Pieck-Strasse 35**
**O-8122 Radebeul(DE)**

(72) Erfinder: **Hagen, Volker, Dr.rer.nat.**
**Kühlungsborner Strasse 29**
**O-1093 Berlin(DE)**
Erfinder: **Reck, Günter**
**Dolgenseestrasse 21**
**O-1136 Berlin(DE)**
Erfinder: **Gentsch, Brigitte**
**Strasse der Befreiung 68**

**O-1136 Berlin(DE)**
Erfinder: **Heidrich, Hans-Joachim, Dipl.Chem.**
**Ebereschenstrasse 32**
**O-8030 Dresden(DE)**
Erfinder: **Jänsch, Hans-Joachim**
**Wilhelm-Pieck-Strasse 292**
**O-8122 Radebeul(DE)**
Erfinder: **Wielop, Ingrid**
**Blumenstrasse 5**
**O-8122 Radebeul(DE)**
Erfinder: **Lohmann, Dieter**
**Hoflössnitzstrasse 30**
**O-8122 Radebeul(DE)**

(74) Vertreter: **Puchberger, Rolf, Dipl. Ing. et al**
**Patentanwälte, Dipl. Ing. Georg Puchberger**
**Dipl. Ing. Rolf Puchberger Dipl. Ing. Peter**
**Puchberger Singerstrasse 13 Postfach 55**
**A-1010 Wien(AT)**

(54) **Kompaktes, kristallines 3-Cyan-2-morpholino-5(pyrid-4-yl)-pyridin hoher Schüttdichte und Verfahren zu seiner Herstellung.**

(57) 3-Cyan-2-morpholino-5-(pyrid-4-yl)-pyridin, hohe Schüttdichte, 3 Kristallmodifikationen, $\alpha$-, $\gamma$- und $\epsilon$-Form, Kardiotonikum

Kompaktes, kristallines 3-Cyan-2-morpholino-5-(pyrid-4-yl)-pyridin hoher Schüttdichte kann dadurch hergestellt werden, daß man dieses entweder

a) in Säuren löst und aus den erhaltenen Lösungen mit Basen ausfällt oder

b) in Chlorkohlenwasserstoffen löst und aus den erhaltenen Lösungen durch Zugabe von aliphatischen Kohlenwasserstoffen ausfällt oder

c) aus aliphatischen Carbonsäureestern umkristallisiert.

Das so erhaltene kompakte, kristalline 3-Cyan-2-morpholino-5-(pyrid-4-yl)-pyridin besitzt eine Schüttdichte von etwa 220 bis 360 g/l und ist für die galenische Verarbeitung sehr gut geeignet.

EP 0 445 553 A1

Die Erfindung betrifft kompaktes, kristallines 3-Cyan-2-morpholino-5-(pyrid-4-yl)-pyridin hoher Schüttdichte und Verfahren zu seiner Herstellung. 3-Cyan-2-morpholino-5-(pyrid-4-yl)-pyridin kann als Kardiotonikum mit gleichzeitig vasodilatatorischen Eigenschaften eingesetzt werden.

Es ist bekannt, 3-Cyan-2-morpholino-5-(pyrid-4-yl)-pyridin durch Umsetzung von 3-Cyan-2-chlor-5-(pyrid-4-yl)-pyridin mit überschüssigem Morpholin herzustellen und das erhaltene Rohprodukt aus Ethanol umzukristallisieren (DD-PS 236 729, Beispiel 7; EP 0 200 024 A2). Dabei fällt das umkristallisierte Produkt in dünnen, filzigen Nadeln an, die nur eine geringe Schüttdichte von 150 g/l aufweisen und die bei der galenischen Verarbeitung erhebliche Schwierigkeiten bereiten.

Es besteht daher ein dringendes Bedürfnis nach einem kompakten, kristallinen 3-Cyan-2-morpholino-5-(pyrid-4-yl)-pyridin hoher Schüttdichte und nach Verfahren, die dessen Herstellung ermöglichen.

Der Erfindung liegt die Aufgabe zugrunde, ein derartiges kompaktes, kristallines 3-Cyan-2-morpholino-5-(pyrid-4-yl)-pyridin mit einer hohen Schüttdichte und Verfahren zu seiner Herstellung aufzufinden.

Erfindungsgemäß wird das dadurch erreicht, daß man 3-Cyan-2-morpholino-5-(pyrid-4-yl)-pyridin entweder

a) in Säuren löst und aus den erhaltenen Lösungen dieses mit Basen ausfällt oder

b) in Chlorkohlenwasserstoffen löst und dieses durch Zugabe von aliphatischen Kohlenwasserstoffen aus seinen Lösungen ausfällt oder

c) aus aliphatischen Carbonsäureestern umkristallisiert.

Entsprechend der vorliegenden Erfindung ist es möglich, das 3-Cyan-2-morpholino-5-(pyrid-4-yl)-pyridin sowohl als Rohprodukt als auch in gereinigtem Zustand einzusetzen.

Eine besondere Ausführungsform der Erfindung besteht darin, die Lösungen des 3-Cyan-2-morpholino-5-(pyrid-4-yl)-pyridin vor dem Ausfällen bzw. Auskristallisieren einer Behandlung mit Adsorbentien wie Aktivkohle oder Kieselgel zu unterwerfen, was insbesondere bei Einsatz eines rohen 3-Cyan-2-morpholino-5-(pyrid-4-yl)-pyridin von Vorteil ist.

Soll das 3-Cyan-2-morpholino-5-(pyrid-4-yl)-pyridin aus seinen sauren Lösungen mit Basen gefällt werden, ist es entsprechend der vorliegenden Erfindung vorteilhaft, dies bei Temperaturen von 15 bis 40 °C, vorzugsweise jedoch bei 30 °C, durchzuführen.

Für die Durchführung des Verfahrens gemäß der Variante a) eignen sich sowohl anorganische als auch organische Säuren, während als Basen Alkalilaugen; Ammoniaklösungen oder Alkalikarbonatlösungen eingesetzt werden können.

Eine besondere Ausführungsform der Erfindung bei der Durchführung des Verfahrens gemäß der Variante a) besteht darin, daß aus den Lösungen des 3-Cyan-2-morpholino-5-(pyrid-4-yl)-pyridins in Mineralsäuren durch Zugabe weiterer Mineralsäure dieses als mineralsaures Salz zunächst ausgefällt, abgetrennt, in Wasser gelöst und gegebenenfalls nach Behandlung mit Adsorbentien mit Basen ausgefällt wird.

Soll das 3-Cyan-2-morpholino-5-(pyrid-4-yl)-pyridin aus Chlorkohlenwasserstoffen mittels aliphatischer Kohlenwasserstoffe nach dem Verfahren gemäß der Variante b) ausgefällt werden, eignen sich hierzu als Chlorkohlenwasserstoffe beispielsweise Chloroform oder Dichlormethan, während als aliphatische Kohlenwasserstoffe vorteilhaft solche mit 5 bis 10 C-Atomen, oder Gemische derselben, zum Beispiel Hexan oder Petrolether: verwendet werden können.

Soll das 3-Cyan-2-morpholino-5-(pyrid-4-yl)-pyridin nach dem Verfahren gemäß Variante c) umkristallisiert werden, besteht eine besondere Ausführungsform der Erfindung darin, daß die Fällung durch schnelles und intensives Abkühlen, insbesondere unter Rühren durchgeführt wird. Als aliphatische Carbonsäureester eignen sich insbesondere solche mit aliphatischen Carbonsäuren mit 1 - 3 C-Atomen und aliphatischen Alkoholen mit 1 - 4 C-Atomen, vorzugsweise jedoch Butylacetat.

Das entsprechend den Verfahren der vorliegenden Erfindung erhaltene kompakte, kristalline 3-Cyan-2-morpholino-5-(pyrid-4-yl)-pyridin fällt je nach den gewählten Reaktionsbedingungen in 3 bisher nicht bekannten kristallinen Modifikationen an, die hier als α-, γ- und ε -Form bezeichnet werden.

Dabei ist keine der 3 Modifikationen einem bestimmten Verfahren a), b) oder c) zuzuordnen. Vielmehr führen selbst geringfügige Veränderungen innerhalb der genannten Verfahren wie Wechsel der Säure und/oder Base im Fall der Variante a) oder Wechsel des chlorierten Kohlenwasserstoffes und/oder des aliphatischen Kohlenwasserstoffes im Fall der Variante b) oder auch nur Änderungen im Kühlprozeß und der Kristallisationsgeschwindigkeit zu unterschiedlichen Kristallmodifikationen oder Gemischen derselben, wie aus den Beispielen zu ersehen ist.

Das ist aber nicht entscheidend. Wichtig ist, daß unabhängig von den erfindungsgemäß anfallenden Kristallmodifikationen bzw. -modifikationsgemischen immer ein kompaktes, kristallines 3-Cyan-2-morpholino-5-(pyrid-4-yl)-pyridin mit einer im Vergleich zum Stand der Technik hohen Schüttdichte von etwa 220 bis 360 g/l erhalten wird, das sich in Kombination mit pharmazeutischen Träger- und Hilfsstoffen gut und in hoher Qualität zu pharmazeutischen Zubereitungen verarbeiten läßt.

2

In den nachfolgenden Tabellen 1 bis 4 sind die zur Identifizierung wichtigen Daten der $\alpha$-, $\gamma$- und $\epsilon$ - Modifikation des 3-Cyan-2-morpholino-5-(pyrid-4-yl)-pyridin beschrieben.

In Tabelle 1 sind die aus Röntgenbeugungsuntersuchungen ermittelten Kristalldaten der $\alpha$- und der $\gamma$-Modifikation dieser Verbindung aufgeführt.

## Tabelle 1: Kristalldaten

| | I-$\alpha$ | I-$\gamma$ |
|---|---|---|
| Bruttoformel | $C_{15}H_{14}N_4O$ | $C_{15}H_{14}N_4O$ |
| Kristallsystem | monoklin | triklin |
| Raumgruppe | $P2_1/n$ | $P\bar{1}$ |
| Gitterkonstanten: a | 19,495 (11) Å | 6,657 (2) Å |
| b | 4,697 (2) Å | 8,420 (2) Å |
| c | 28,509 (26) Å | 12,067 (5) Å |
| $\alpha$ | 90° | 103,64 (3) ° |
| $\beta$ | 92,01 (7)° | 99,11 (2)° |
| $\gamma$ | 90° | 102,57 (2)° |
| Zahl der Formeleinheiten in der Elementarzelle | 8 | 2 |
| $D_{calc.}$ | 1,356 gcm$^{-3}$ | 1,325 gcm$^{-3}$ |

Abbildung 1 zeigt das Molekül von I mit der Numerierung der Atome.

Abb. 1

Die Tabellen 2 und 3 enthalten die Atomkoordinaten von I-$\alpha$ und I-$\gamma$ . In Tabelle 4 sind die aus den Einkristallstrukturdaten berechneten Pulverdiagramme (d-Werte und relative Intensitäten) von I-$\alpha$ und I-$\gamma$ sowie das mittels Guinier-Technik ermittelte experimentelle Pulverdiagramm von I-$\epsilon$ dargestellt.

Tabelle 2: <u>Atomkoordinaten ($\times 10^4$) von I-$\alpha$</u>

| | Molekül A | | | | Molekül B | | |
|---|---|---|---|---|---|---|---|
| Atom | x/a | y/b | z/c | Atom | x/a | y/b | z/c |
| O18 | 1072(2) | 6942(10) | 2563(2) | O18 | 3607(2) | -3040(10) | 1030(2) |
| N6 | 1990(2) | 2304(10) | 1254(2) | N6 | 5964(3) | 5622(10) | 1732(2) |
| N10 | 2245(3) | -5036(10) | -5036(2) | N10 | 9203(3) | -0442(10) | 1959(2) |
| N14 | -40(3) | 7009(10) | 649(2) | N14 | 5005(4) | -10510(20) | 249(3) |
| N15 | 1296(3) | 5390(10) | 1626(2) | N15 | 5034(3) | -5090(10 | 1232(2) |
| C1 | 1429(3) | 3010(10) | 1240(2) | C1 | 5609(3) | -6590(10) | 1325(2) |
| C2 | 967(3) | 3665(10) | 045(2) | C2 | 6130(3) | -0222(10) | 1032(2) |
| C3 | 1142(3) | 1033(10) | 477(2) | C3 | 6703(3) | -0746(10) | 1177(2) |
| C4 | 1724(3) | 203(10) | 500(2) | C4 | 7066(3) | -7673(10) | 1503(2) |
| C5 | 2153(3) | 596(10) | 096(2) | C5 | 6606(3) | -6161(20) | 1057(2) |
| C7 | 1913(3) | -1014(10) | 127(2) | C7 | 7700(3) | -0019(10) | 1730(2) |
| C0 | 1449(3) | -2592(10) | -227(2) | C0 | 0222(3) | -9004(20) | 1491(2) |
| C9 | 1654(3) | -4549(10) | -563(2) | C9 | 0007(4) | -9943(20) | 1619(3) |
| C11 | 2710(3) | -5034(20) | -229(2) | C11 | 0770(4) | -6769(20) | 2201(3) |
| C12 | 2544(4) | -3070(10) | 122(2) | C12 | 0097(3) | -6464(20) | 2090(2) |
| C13 | 413(3) | 5536(10) | 756(2) | C13 | 5913(4) | -9402(20) | 590(3) |
| C16 | 664(3) | 6593(20) | 1762(2) | C16 | 4714(4) | -5940(20) | 766(3) |
| C17 | 526(3) | 6019(20) | 2263(2) | C17 | 3994(4) | -5914(20) | 755(3) |
| C19 | 1667(3) | 5520(20) | 2441(3) | C19 | 3940(4) | -4054(20) | 1491(3) |
| C20 | 1073(3) | 6195(10) | 1959(2) | C20 | 4691(3) | -3912(20) | 1539(2) |

## Tabelle 3: <u>Atomkoordinaten $(\times 10^4)$ von I-γ</u>

| | Molekül A | | | | Molekül B | | |
|---|---|---|---|---|---|---|---|
| Atom | x/a | y/b | z/c | Atom | x/a | y/b | z/c |
| O18 | 3544(3) | -7776(2) | 63(1) | C7 | 6578(3) | 685(2) | 6308(1) |
| N6 | 6737(2) | -3377(2) | 3553(1) | C8 | 5130(3) | -191(3) | 6883(2) |
| N10 | 7022(3) | 1869(2) | 8325(1) | C9 | 5813(4) | 1063(3) | 7870(2) |
| N14 | -270(3) | -6504(3) | 3528(2) | C11 | 9208(4) | 1392(3) | 7770(2) |
| N15 | 4319(2) | -5482(2) | 2162(1) | C12 | 8687(3) | 144(3) | 6783(2) |
| C1 | 4762(3) | -4386(2) | 3185(1) | C13 | 1304(3) | -5538(2) | 3656(2) |
| C2 | 3311(3) | -4322(2) | 3881(1) | C16 | 2236(3) | -6032(3) | 1414(5) |
| C3 | 3906(3) | -3112(2) | 4894(1) | C17 | 1946(4) | -7774(3) | 678(2) |
| C4 | 5909(3) | -2016(2) | 5246(1) | C19 | 5560(4) | -7284(3) | 791(2) |
| C5 | 7255(3) | -2272(3) | 4538(1) | C20 | 6002(3) | -5535(3) | 1553(2) |

## Tabelle 4: <u>Experimentelle und berechnete Pulverdiagramme</u>

| I-α | | I-γ | | I-ε | |
|---|---|---|---|---|---|
| d(Å) | Intens. | d(Å) | Intens. | d(Å) | Intens. |
| 14.24 | 22 | 12.20 | 42 | 9.36 | 30 |
| 9.74 | 14 | 7.89 | 11 | 7.77 | 62 |
| 8.66 | 9 | 6.33 | 64 | 7.12 | 41 |
| 8.18 | 22 | 6.21 | 41 | 6.50 | 100 |
| 7.91 | 84 | 5.74 | 18 | 5.78 | 73 |
| 7.12 | 43 | 5.17 | 20 | 5.41 | 12 |
| 6.38 | 10 | 4.62 | 5 | 5.24 | 61 |
| 6.28 | 100 | 4.25 | 32 | 4.64 | 40 |
| 5.66 | 21 | 4.17 | 13 | 4.23 | 68 |
| 5.52 | 55 | 4.11 | 23 | 4.13 | 23 |
| 5.43 | 10 | 3.84 | 67 | 4.03 | 10 |
| 4.87 | 43 | 3.82 | 16 | 3.96 | 11 |
| 4.64 | 20 | 3.59 | 10 | 3.88 | 85 |
| 4.56 | 14 | 3.47 | 100 | 3.80 | 14 |
| 4.50 | 20 | 3.33 | 8 | 3.66 | 43 |
| 4.46 | 27 | 3.15 | 14 | 3.58 | 22 |
| 4.35 | 8 | | | 3.50 | 17 |
| 4.21 | 22 | | | 3.42 | 80 |
| 4.12 | 9 | | | 3.31 | 41 |
| 4.07 | 18 | | | | |
| 4.01 | 71 | | | | |
| 3.88 | 55 | | | | |
| 3.83 | 33 | | | | |
| 3.70 | 30 | | | | |
| 3.66 | 43 | | | | |
| 3.61 | 15 | | | | |
| 3.58 | 9 | | | | |
| 3.56 | 30 | | | | |
| 3.48 | 6 | | | | |
| 3.42 | 34 | | | | |

Beispiel 1

a) 3-Cyan-2-morpholino-5-(pyrid-4-yl)-pyridin-Hydrochlorid-Monohydrat

5,0 kg 3-Cyan-2-morpholino-5-(pyrid-4-yl)-pyridin werden mit 25 l Wasser und 2,0 l technischer Salzsäure vermischt und auf ca. 50 °C erhitzt. Zu der gelben Lösung werden 3,0 l Salzsäure gegeben und der Ansatz abgekühlt. Nach Kristallisation rührt man noch 2 Stunden bei Temperaturen unterhalb 20 °C. Man zentrifugiert und wäscht mit einem Gemisch aus 5,4 l Wasser und 0,6 l Salzsäure.
Ausbeute: 5,3 kg (88 % der Theorie).

b) 3-Cyan-2-morpholino-5-(pyrid-4-yl)-pyridin ($\gamma$-Modifikation)

5,3 kg 3-Cyan-2-morpholino-5-(pyrid-4-yl)-pyridin-Hydrochlorid-Monohydrat werden in 40 l Wasser suspendiert, die Suspension auf 50 °C erwärmt, 0,5 kg Aktivkohle zugesetzt, nach 15 Minuten Rühren abgesaugt und mit Wasser gewaschen. Das Filtrat kühlt man auf 30 °C ab und läßt unter Rühren innerhalb einer Stunde bei dieser Temperatur ca. 2.7 l konzentrierte Natronlauge zulaufen bis ein pH-Wert von 8 - 9 erreicht ist. Es wird zentrifugiert und mit insgesamt 30 l Wasser gewaschen.
Ausbeute: 4,1 kg (93,2 % der Theorie).
F.: 126 - 128 °C.
Schüttdichte der gemörserten Probe: ca. 360 g/l.

Beispiel 2

3-Cyan-2-morpholino-5-(pyrid-4-yl)-pyridin ($\gamma$-Modifikation)

1 g gereinigtes 3-Cyan-2-morpholino-5-(pyrid-4-yl)-pyridin beliebiger Modifikation werden in 4 ml 1N HCl unter Erwärmen auf ca. 50 °C gelöst und unter Rühren bei 30 - 40 °C durch Versetzen mit ca. 4 ml 1N NaOH gefällt. Es wird abgesaugt mit Wasser gewaschen und bei 110 °C getrocknet.
Ausbeute: 0,95 g (95 % der Theorie).
F.: 126 - 128 °C.
Schüttdichte der gemörserten Probe: ca. 360 g/l

Beispiel 3

3-Cyan-2-morpholino-5-(pyrid-4-yl)-pyridin ($\alpha$-Modifikation)

4,0 kg vorgereinigtes 3-Cyan-2-morpholino-5-(pyrid-4-yl)-pyridin werden in 40 l Wasser suspendiert und 5,5 kg Milchsäure zugegeben. Man erhitzt auf 50 °C, gibt 0,2 kg Aktivkohle zu und filtriert nach 15minutigem Rühren. Es wird mit warmem Wasser gewaschen, auf 30 °C gekühlt und ca. 1,25 l konzentrierte Natronlauge bis zum Erreichen eines pH-Wertes von 8 - 9 zugetropft. Man rührt noch 1 Stunde, zentrifugiert wäscht mit insgesamt 30 l Wasser und trocknet.
Ausbeute: 3,74 kg (93,5 % der Theorie), schwach gelbliche Kristalle.
F.: 127 - 129 °C.
Schüttdichte der gemörserten Probe: ca. 230 g/1

Beispiel 4

3-Cyan-2-morpholino-5-(pyrid-4-yl)-pyridin ($\gamma$- und $\epsilon$-Modifikation)

100 g vorgereinigtes 3-Cyan-2-morpholino-5-(pyrid-4-yl)-pyridin werden in 1,2 l Wasser suspendiert, dem 39 ml technische Salzsäure und 5 g Aktivkohle zugesetzt werden. Es wird auf 50 °C erwärmt und filtriert. Zum Filtrat tropft man 26 ml konzentrierte Ammoniaklösung bei ca. 30 °C. rührt noch 1 Stunde, saugt ab und wäscht noch 6 mal mit 50 ml Wasser.
Ausbeute: 96,2 g (96,2 % der Theorie).
F. : (124) 127 - 128 °C.
Schüttdichte der gemörserten Probe: ca. 300 g/l.

Beispiel 5

3-Cyan-2-morpholino-5-(pyrid-4-yl)-pyridin ($\alpha$-Modifiaktion)

3 g gereinigtes 3-Cyan-2-morpholino-5-(pyrid-4-yl)-pyridin beliebiger Modifikation werden in 5 ml CHCl$_3$

unter leichtem Erwärmen gelöst und unter Rühren durch Versetzen mit 10 ml Hexan gefällt. Es wird abgesaugt und getrocknet.
Ausbeute: 2,82 g (94 % der Theorie).
F.: 127 - 128 °C.
Schüttdichte der gemörserten Probe: ca. 230 g/l.

Beispiel 6

3-Cyan-2-morpholino-5-(pyrid-4-yl)-pyridin ($\epsilon$-Modifikation)

1 g gereinigtes 3-Cyan-2-morpholino-5-(pyrid-4-yl)-pyridin beliebiger Modifikation werden in 4 ml $CH_2Cl_2$ unter Erwärmen gelöst und unter Rühren durch Versetzen mit 5 ml Hexan gefällt. Es wird abgesaugt und getrocknet.
Ausbeute: 0,97 g (97 % der Theorie).
F.: (124) 127 - 130 °C.
Schüttdichte der gemörserten Probe: ca. 220 g/l

Beispiel 7

3-Cyan-2-morpholino-5-(pyrid-4-yl)-pyridin (Gemisch aus $\alpha$- und $\epsilon$-Modifikation)

125 g Rohprodukt von 3-Cyan-2-morpholino-5-(pyrid-4-yl)-pyridin werden mit 1 l Butylacetat bis zur Auflösung unter Rückfluß gekocht. Es wird heiß filtriert und die Lösung 10 Minuten mit 10 g Aktivkohle unter Erwärmen gerührt. Es wird heiß abgesaugt. unter intensivem Rühren mit Eiswasser gekühlt, abgesaugt, mit Butylacetat und Methanol gewaschen, erneut in 900 ml Butylacetat unter Erwärmen gelöst, mit 7 g Aktivkohle behandelt, filtriert und die ca. 70 °C warme Lösung durch intensives Rühren und Kühlen zur Kristallisation gebracht. Anschließend wird 2 Stunden bei Raumtemperatur nachgerührt, über Nacht im Kühlschrank stehen gelassen, abgesaugt, mit Methanol gewaschen und bei 110 °C getrocknet.
Ausbeute: 80 g (67 % der Theorie).
F.: (124) 128 - 130 °C.
Schüttdichte der gemörserten Probe: ca. 245 g/l
Durch Einengen der Mutterlaugen und Umkristallisation des hierbei erhaltenen Produktes auf die beschriebene Weise läßt sich die Ausbeute erhöhen.

Beispiel 8

3-Cyan-2-morpholino-5-(pyrid-4-yl)-pyridin (Gemisch aus $\gamma$- und $\epsilon$-Modifikation)

Es wird analog Beispiel 5 gearbeitet, jedoch erfolgt das Fällen durch Zutropfen von 60 ml einer 45 %igen $K_2CO_3$-Lösung bei 30 °C.
Ausbeute: 46,6 g (93.2 % der Theorie).
F.: 121 - 123 und 126 - 128 °C.
Schüttdichte der gemörserten Probe: ca. 330 g/l.

**Patentansprüche**

1. Kompaktes, kristallines 3-Cyan-2-morpholino-5-(pyrid-4-yl)-pyridin hoher Schüttdichte.

2. Kompaktes, kristallines 3-Cyan-2-morpholino-5-(pyrid-4-yl)-pyridin gemäß Anspruch 1 mit einer Schüttdichte von etwa 220 bis 360 g/l.

3. Verfahren zur Herstellung von kompaktem, kristallinen 3-Cyan-2-morpholino-5-(pyrid-4-yl)pyridin gemäß den Ansprüchen 1 und 2, dadurch gekennzeichnet, daß man 3-Cyan-2-morpholino-5-(pyrid-4-yl)-pyridin entweder
   a) in Säuren löst und aus den erhaltenen Lösungen dieses mit Basen ausfällt oder
   b) in Chlorkohlenwasserstoffen löst und durch Zugabe von aliphatischen Kohlenwasserstoffen ausfällt oder
   c) aus aliphatischen Carbonsäureestern umkristallisiert.

4. Verfahren nach Anspruch 3, dadurch gekennzeichnet. daß rohes 3-Cyan-2-morpholino-5-(pyrid-4-yl)-pyridin eingesetzt wird.

5. Verfahren nach den Ansprüchen 3 und 4, dadurch gekennzeichnet, daß erhaltene Lösungen des 3-Cyan-2-morpholino-5-(pyrid-4-yl)-pyridin mit Adsorbentien wie Aktivkohle oder Kieselgel behandelt werden.

6. Verfahren nach den Ansprüchen 3a, 4 und 5, dadurch gekennzeichnet, daß das 3-Cyan-2-morpholino-5-(pyrid-4-yl)-pyridin aus seinen sauren Lösungen bei Temperaturen von 15 bis 40 °C, vorzugsweise bei ca. 30 °C, mit Basen gefällt wird.

7. Verfahren nach den Ansprüchen 3a bis 6, dadurch gekennzeichnet, daß als Säuren anorganische oder organische Säuren eingesetzt werden.

8. Verfahren nach den Ansprüchen 3a bis 7, dadurch gekennzeichnet, daß als Basen Alkalilaugen, Ammoniaklösungen oder Alkalikarbonatlösungen eingesetzt werden.

9. Verfahren nach den Ansprüchen 3a bis 8, dadurch gekennzeichnet, daß aus den Lösungen von 3-Cyan-2-morpholino-5-(pyrid-4-yl)-pyridinin Mineralsäuren durch Zugabe weiterer Mineralsäure dieses als mineralsaures Salz gefällt, abgetrennt, in Wasser gelöst und gegebenenfalls nach Behandlung mit Adsorbentien mit Basen gefällt wird.

10. Verfahren nach den Ansprüchen 3b, 4 und 5, dadurch gekennzeichnet, daß als aliphatische Kohlenwasserstoffe solche mit 5 bis 10 C-Atomen oder Gemische derselben eingesetzt werden.

11. Verfahren nach den Ansprüchen 3b, 4, 5 und 10, dadurch gekennzeichnet, daß als Chlorkohlenwasserstoffe aliphatische Chlorkohlenwasserstoffe wie Dichlormethan oder Chloroform eingesetzt werden.

12. Verfahren nach den Ansprüchen 3c, 4 und 5. dadurch gekennzeichnet, daß als aliphatische Carbonsäureester solche aus aliphatischen Carbonsäuren mit 1 bis 3 C-Atomen und aliphatischen Alkoholen mit 1 bis 4 C-Atomen, vorzugsweise Butylacetat, verwendet werden.

13. Verfahren nach den Ansprüchen 3c, 4, 5 und 12, dadurch gekennzeichnet, daß die Umkristallisation unter schnellem und intensivem Abkühlen und unter Rühren durchgeführt wird.

Europäisches Patentamt

**EUROPÄISCHER TEILRECHERCHENBERICHT,**
der nach Regel 45 des Europäischen Patentübereinkommens für das weitere Verfahren als
europäischer Recherchenbericht gilt

Nummer der Anmeldung

## EINSCHLÄGIGE DOKUMENTE

EP 91101973.5

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| Y | DD - A1 - 256 131 <br> (AK. DER WISSENSCHAFTEN) <br> * Anspruch 1 * <br> -- | 1,3-5 | C 07 D 213/85 |
| D,Y | DD - A1 - 236 729 <br> (AK. DER WISSENSCHAFTEN) <br> * Ansprüche 1,2; Seite 2, 2. Absatz * <br> -- | 1,3-5 | |
| Y | DD - A1 - 237 657 <br> (AK. DER WISSENSCHAFTEN) <br> * Ansprüche 1,2; Seite 2, 1. Absatz * <br> ---- | 1,3-5 | |

RECHERCHIERTE SACHGEBIETE (Int. Cl.5)

C 07 D 213/00

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich
ist, auf der Grundlage einiger Patentansprüche sinnvolle Ermittlungen über den Stand der Technik
durchzuführen.

Vollständig recherchierte Patentansprüche: *2-13*

Unvollständig recherchierte Patentansprüche: –

Nicht recherchierte Patentansprüche: *1*

Grund für die Beschränkung der Recherche:

*Unklarheiten (Dichte und Kristall nicht definiert)*

| Recherchenort <br> WIEN | Abschlußdatum der Recherche <br> 10-05-1991 | Prüfer <br> HOCHHAUSER |
|---|---|---|

EPA Form 1505.1  03.82